# EUROPEAN PATENT SPECIFICATION

(11) **EP 2 709 640 B1**
(45) Date of publication and mention of the grant of the patent: **25.11.2015**
(21) Application number: 12722358.4
(22) Date of filing: 18.05.2012
(51) Int. Cl.: A61K 36/064, A61K 36/899, A61P 29/00

(54) **USE OF FERMENTED WHEAT GERM IN THE TREATMENT OF INFLAMMATORY BOWEL DISEASE**
VERWENDUNG VON FERMENTIERTEM WEIZENKEIM BEI DER BEHANDLUNG VON ENTZÜNDLICHER DARMERKRANKUNG
UTILISATION DE GERME DE BLÉ FERMENTÉ DANS LE TRAITEMENT DE LA MALADIE INTESTINALE INFLAMMATOIRE

(30) Priority: 20.05.2011 GB 201108560
(43) Date of publication of application: 26.03.2014
(73) Proprietor: 3CH Ltd, 7002 Chur (CH)
(72) Inventor: RASO, Erzsebet, CH-7002 Chur (CH); LAPIS, Karoly, CH-7002 Chur (CH)
(74) Representative: Hart, Deborah Mary
(86) International application number: PCT/EP2012/059261
(87) International publication number: WO 2012/159988

(56) References cited:
- WO-A1-2012/018370
- WO-A2-2005/019429
- WO-A2-2010/100515
- US-A1- 2005 266 106
- US-A1- 2008 044 402
- TELEKES ANDRAS AND HIDVEGI MATE: "Avemar's effect mechanism (part2) - immunological effects", NOGYOGYASZATI ONKOLOGIA,, vol. 6, 1 January 2001 (2001-01-01), pages 1-4,40, XP003016918,
- TELEKES ANDRAS ET AL: "Fermented wheat germ extract (avemar) inhibits adjuvant arthritis", NEW YORK ACADEMY OF SCIENCES. ANNALS, WILEY-BLACKWELL PUBLISHING, INC, US, vol. 1110, 1 September 2007 (2007-09-01), pages 348-361, XP009160276, ISSN: 0077-8923, DOI: 10.1196/ANNALS.1423.037 [retrieved on 2007-09-27]
- BEN-ARYE E ET AL: "Wheat grass juice in the treatment of active distal ulcerative colitis: A randomized double-blind placebo-controlled trial", SCANDINAVIAN JOURNAL OF GASTROENTEROLOGY, vol. 37, no. 4, April 2002 (2002-04), pages 444-449, XP009160311, ISSN: 0036-5521
- SATYAVATI RANA, JASPREET KAUR KAMBOJ, VANDANA GANDHI: "Living life the natural way - Wheatgrass and health", FUNCTIONAL FOODS IN HEALTH AND DISEASE, vol. 11, 30 November 2011 (2011-11-30), pages 444-456, XP002677892,
- SHAH K V ET AL: "Anti-ulcer activity of triticum aestivum on ethanol induced mucosal damage (cytoprotective activity) in wistar rats", PHARMACOLOGYONLINE 2011 UNIVERSITY OF SALERNO ITA, vol. 2, 2011, pages 929-935, XP009160314, ISSN: 1827-8620
- BROWN L ET AL: "Fermented wheat germ extract (avemar) in the treatment of cardiac remodeling and metabolic symptoms in rats", EVIDENCE-BASED COMPLEMENTARY AND ALTERNATIVE MEDICINE 2011 HINDAWI PUBLISHING CORPORATION GBR LNKD- DOI:10.1093/ECAM/NEP090, vol. 2011, 2011, XP009160264, ISSN: 1741-427X

## Description

The present application relates to the new use of a known fermented wheat germ product in the treatment of inflammatory bowel disease.

WO 99/08694 describes a product obtained by the fermentation of wheat germ with Baker's yeast (*Saccharomyces cerevisiae*) which was found to have an immunostimulatory and metastasis inhibiting effect. The product contains 0.12-0.52mg/g dry material 2,6,- dimethoxy-p-benzoquinone (2.6-DMBQ). This product, known commercially as Avemar™ has also been used as an anti-inflammatory agent for preventing or treating or alleviating inflammatory conditions in particular rheumatoid arthritis as described in WO 2004/014406. The product was found to increase levels of cytokines including TNFα. WO 2004/014146 also describes the use of this product as a dietary supplement for animals. It provides quicker gain in body weight as well as improving resistance to infectious diseases.

General treatments for inflammatory bowel disease include immunosuppression such as administering antagonists of TNFα. The fermented wheat germ product has been previously shown to elevate levels of TNFα during treatment of rheumatoid arthirits. However it has now surprisingly been found to be effective at treating inflammatory bowel disease.

Thus in a first aspect the present invention provides a fermented dried material derived from the fermented liquid obtainable by fermenting wheatgerm with *Saccharomyces cerevisiae* in an aqueous medium or fractions thereof for use in the treatment of inflammatory bowel disease.

As used herein "inflammatory bowel disease" (IBD) relates to inflammatory conditions of the colon and small intestine. These include Crohn's disease, ulcerative colitis, Collagenous colitis, Lymphocytic colitis, Ischaemic colitis, Diversion colitis, Beliefs disease and Indeterminate colitis.

The fermented dried material is obtainable by the methods described in WO 99/008694 or fractions thereof such as those decribed in WO2010/100515. Typically, the wheategerm is fermented with Baker's yeast (*Saccharomyces cerevisiae*) in water, for about 1 0-24 hours, preferably 15-20 hours or about 18 hours. Other strains of yeast such as *Saccharomyces bayanus* and *Saccharomyces boular,* and other microorganisms used in the fermentation of foods can also be used. The fermentation is carried out at about 25 - 35°C, preferably about 30°C. The weight ratio of the wheat germ and the yeast is 4:1 - 2: 1, preferably about 3:1. The weight ratio of the dry matter and water is 1:6-1:12, preferably about 1:9. After fermentation the solids are removed by a suitable e,g centrifuging for 5-15 minutes at 2000 - 4500 I min, preferably about 3000 I min. The liquid portion is then dried e.g it is boiled, cooled and dried in an appropriate manner. e. g. by lyophilisation or spray-drying. The product is sold commercially under the trade name Avemar™. The product is characterised in that it contains 0.12-0.52mg/g dry material 2,6,-dimethoxy-p-benzoquinone (2.6-DMBQ). The HPLC chromatogram of the product is shown in WO 99/008694.

Alternatively fractions of the dried fermented wheatgerm product obtainable by the methods described in WO 99/008694 can be used. These include the fractions described in WO2010/100515. The dried material is typically dissolved in alcohol, prefereably methanol or ethanol, and then washed and filtered with alcohol. This process can be repeated with the filtrate until the alcoholic phase becomes colorless. The alcoholic phases are united and evaporated to form a fraction known as A2 in WO2010/100515. This is a material mixture. The filtrate (designated fraction Al in WO2010/100515) can be suspended in water, centrifuged, the supernatant decanted, and organic solvent added. The organic solvent is preferably hexane, ethylacetate, or an alcohol, such as methanol or ethanol. The precipitate can be filtered, and dried. The thus produced fraction was called fraction E in WO2010/100515 and contained 15-25% of the starting dry material. Fraction E can be dissolved in water, filtered, and a) if desired, the solution evaporated to dryness (fraction ES), or b) the solution can be gel-chromatographed, and the material remaining in column can be eluted and lyophilized (fraction L). Carbohydrate based gels, preferably agarose-based ones, more preferably, agarose-dextran based gel-filtration materials can be used for gel-filtration chromatography. The column can be washed with as an eluent, diluted acid, preferably, hydrochloric acid, formic acid, acetic acid, apple-vinegar, wine-vinegar, trifluoro-acetic acid, citric acid, tartaric acid, malic acid, ascorbic acid, preferably, 0.1 N hydrochloric acid, or bases, preferably, alkali hydroxides, alkaline earth hydroxides-, oxides, ammonium hydroxide.. Provided the acid or base eluent can not be evaporated, the solution can be neutralized by the use of the appropriate acid or base, and the salt precipitated can be removed, using suitable methods known to the person skilled in the art. The eluted fraction L can be dried by vacuum drying, or lyophilisation, preferably, by lyophilisation.

The fermented dried material of the invention is formulated as a pharmaceutical composition which may also contain one or more excipients, diluents or carriers. Preferably the composition further comprises malodextrin.

The compositions may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or nonaqueous liquids; edible foams or whips; or oil-in-water liquid emulsions or water-in-oil liquid emulsions.

Pharmaceutical formulations adapted for topical administration may be formulated as ointments, creams, suspensions, lotions, powders, solutions, pastes, gels, sprays, aerosols or oils.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for parenteral administration include aqueous and nonaqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may also include other agents conventional in the art having regard to the type of formulation in question, for example those suitable for oral administration may include flavouring agents.

Preferred unit dosage formulations are those containing a daily dose or sub-dose, or an appropriate fraction thereof, of an active ingredient. Suitable daily dosages ate between 0.001-100g, preferably 0.01-50g, more preferably 0.1-40g per kg body weight.

Also described is a method of treating and/or preventing an inflammatory bowel disease comprising administering the fermented dried material derived from the fermented liquid obtainable by fermenting wheatgerm with *Saccharomyces cerevisiae* in an aqueous medium to a subject in need thereof.

Also described is the use of the fermented dried material derived from the fermented liquid obtainable by fermenting wheatgerm with *Saccharomyces cerevisiae* in an aqueous medium in the manufacture of a medicament for use in the treatment of an inflammatory bowel disease.

### EXAMPLES

The examples refer to the following figures
Figure 1 shows the effects of tested compounds on weight loss of animals after 3 days of TNBS treatment.
Figure 2 shows the percentage of the inflamed section of the intestines in proportion to the whole intestinal segment.
Figure 3 shows the severity of the inflammation of the mucus membrane.
Figure 4 compares the weight of the intestines following treatment.
Figure 5 shows the levels of TNF-α after treatment.
Figure 6 shows the Levels of Myeloperoxidase (mU/g).

### METHOD

| *Name of group* | *Treatment* |
|---|---|
| Abs. control | Untreated group |
| 2,4,6 TNBS | 2,4,6 TNBS: 10 mg/ rat |
| Avemar | 2,4,6 TNBS + Avemar treated group 3 g/ kg/ day p.o. treatment |
| Infliximab | 2,4,6 TINBS + infliximab 3,0 mg/ kg/day |
| SASP | 2,4,6 TNBS + Sulfasalazine 50 mg/ kg/day |

Rats were treated with Avemar pulvis for 10 days, then inflammatory bowel disease was induced by injecting TNBS (2,4,6-trinitrobenzenesulfonic acid) in solution. Infliximab was given on day -10, -6, - 3 and day zero prior to TNBS treatment. During the induction method TNBS (10 mg/ rat) was introduced after 12 hours starving in aether-anaesthesia into the colon. The method is the worldwide accepted model for inducing and studying inflammatory bowel disease (Induction of TNBS colitis in mice Scheiffele F, Fuss IJ. Curr Protoc Immunol. 2002 Aug;Chapter 15).

Avemar treatment was continued for additional three days then the animals were sacrificed by cervalis dislocation and the effects of Avemar were investigated and six parameters were explored:
1. The weight loss of the animals between the TNBS induction and exitus. Because of the severe inflammation caused by TNBS the animals loose significant weight. If Avemar reduces the level of inflammation the expected loss of weight should be smaller.
2. The percentage of the inflamed section of the intestines in proportion to the whole intestinal segment. TNBS should destroy the mucus membrane of the intestines but if Avemar is effective, the area of the damaged bowel surface, the lesion should be smaller.
3. The severity of the inflammation of the mucus membrane. Similarly to 2) if Avemar is effective the inflammation should be less severe as well. The scale was measured from 0-11, where 0= no inflammation and 11 = the inflammatory ulcer is at least 7 cms long.
4. The weight of the bowel. The TNBS induced inflammatory bowel disease is associated with the swelling of the intestines, thus growing in weight. If Avemar is effective, the swelling should be less pronounced resulting in smaller weight.
5. TNF-alpha level. According to the current state of the art treatment of IBD TNF-alpha enzyme inhibitors should be used, because the level of TNF-alpha is inversly associated with the severity of the disease. If Avemar is an effective treatment it is expected that Avemar also reduces the TNF-alpha level in the tissue.
6. Myeloperoxidase level in intestinal tissues. As a normal process during inflammation, neutrophil granulocytes migrate into the bowel. Myeloperoxidase is an enzyme found in granulocytes, therefore the abundance of myeloperoxidase enzyme activity is in correlation with the presence of granulocytes, thus with the level of inflammation. The reduced myeloperoxidase levels should indicate that Avemar reduced the level of inflammation and the migration of granulocytes into the bowel was not that profound.

### Results

Every difference with Avemar pulvis and tablet were significant (p < 0.05) versus TNBS treated animals. No significant difference could be measured between Avemar (pulvis or tablet) and Infliximab.

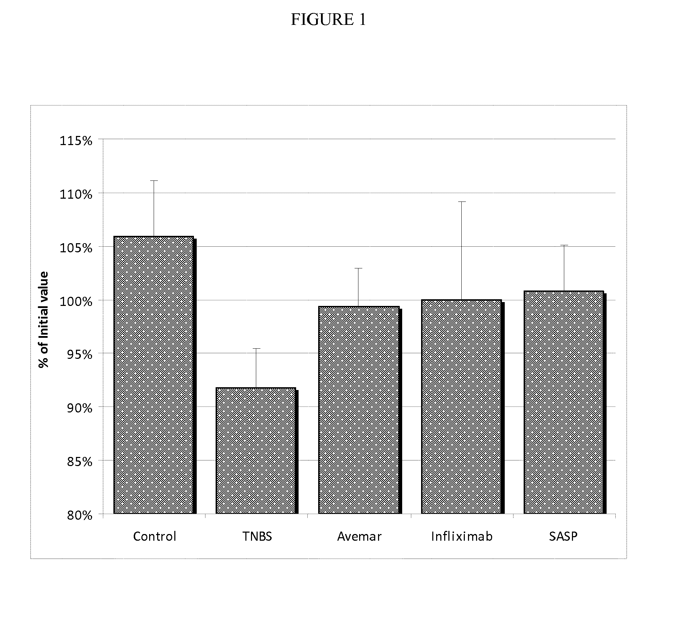

Figure 1 shows the weight loss of the animals between the TNBS induction and exitus, 3 days after TNBS treatment. Avemar (both pulvis and tablet form) could significantly prevent loss of weight, comparable to Infliximab.

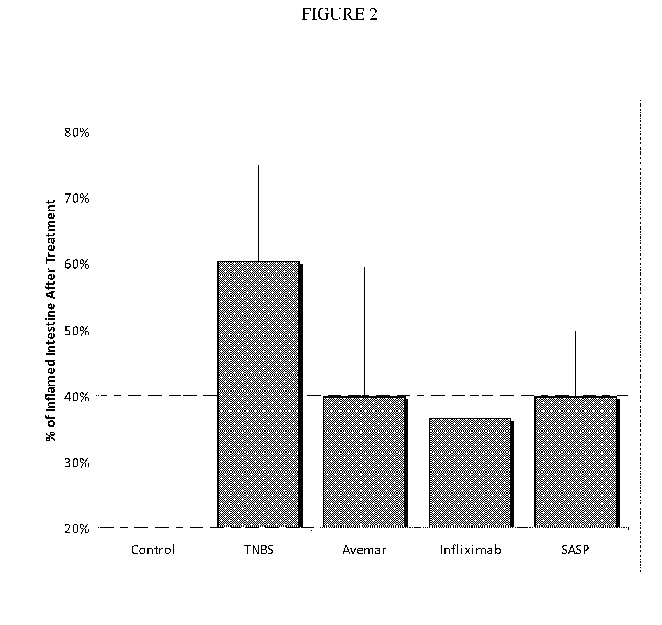

The percentage of the inflamed section of the intestines in proportion to the whole intestinal segment is shown in Figure 2. Avemar (both pulvis and tablet) could significantly reduce the area of inflammation within the effected bowel segment. There was no significant difference between the effects of Avemar and Infliximab.

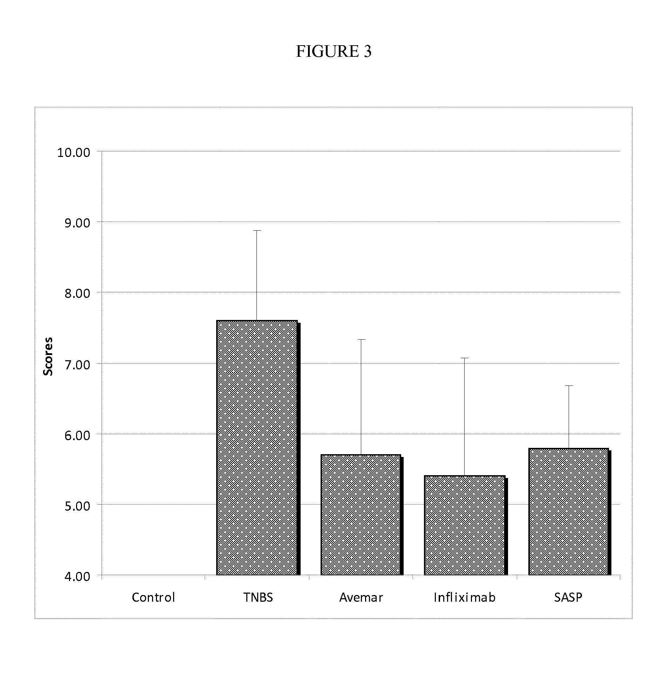

Figure 3 shows the severity of the inflammation of the mucus membrane. Avemar (both pulvis and tablet) could significantly reduce the severity of inflammation within the effected bowel segment. There was no significant difference between the effects of Avemar and Infliximab.

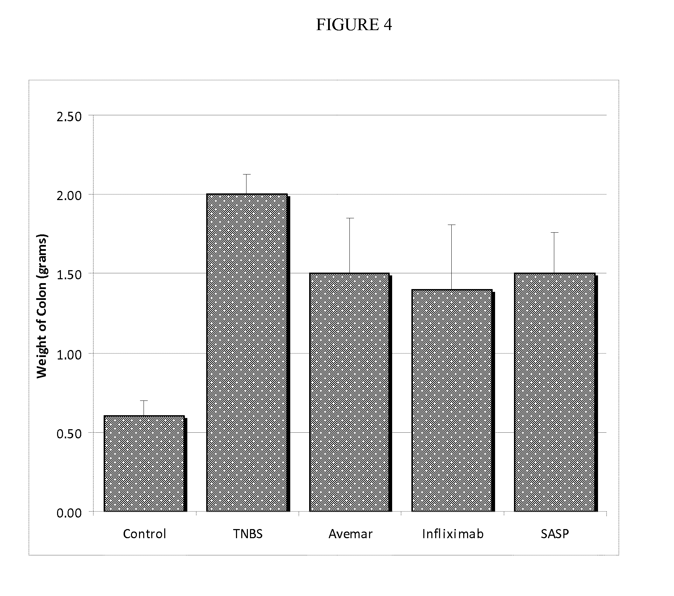

The change in the weight of the intestines is shown in Figure 4. Avemar (both pulvis and tablet) could significantly reduce the weight of the bowel, thus reducing the level of inflammation. There was no significant difference between the effects of Avemar and Infliximab.

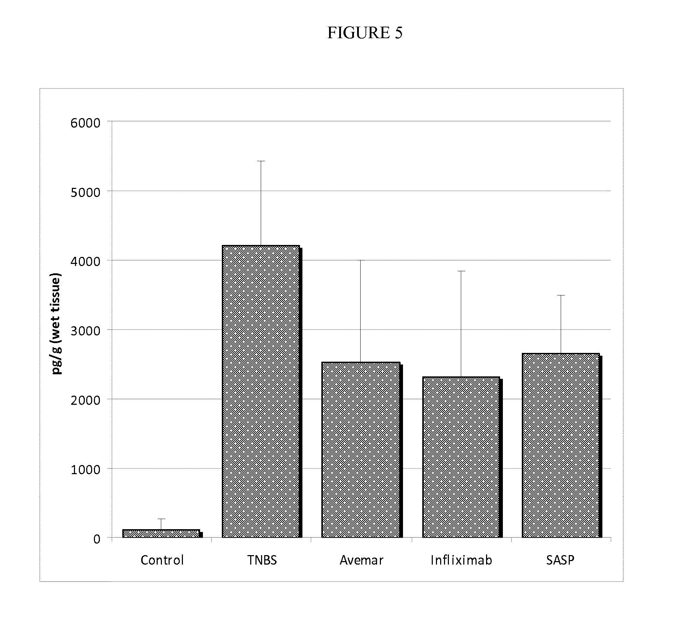

The levels of TNF-α following treatment are shown in Figure 5. Avemar (both pulvis and tablet) could effectively inhibit TNF-alpha comparable to Infliximab. There was no significant difference between the effects of Avemar tablet and Infliximab.

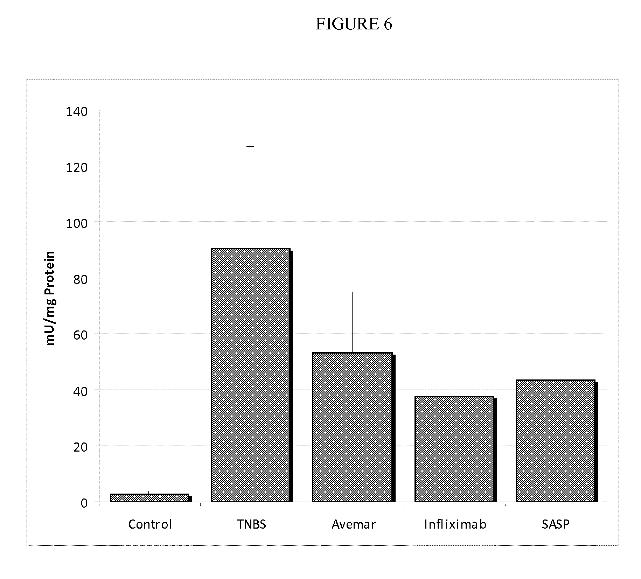

Figure 6 shows the Levels of Myeloperoxidase. Avemar (both pulvis and tablet) could reduce the level of myeloperoxidase indicating the significant reduction of granulocyte infiltration into the mucus membrane of the bowel. There was no significant difference between the effects of Avemar and Infliximab.

## Claims

1. A fermented dried material derived from the fermented liquid obtainable by fermenting wheatgerm with *Saccharomyces cerevisiae* in an aqueous medium or fractions thereof for use in the treatment of inflammatory bowel disease.

2. The fermented dried material of claim 1 wherein said inflammatory bowel disease is selected from Crohn's disease, ulcerative colitis, Collagenous colitis, Lymphocytic colitis, Ischaemic colitis, Diversion colitis, Behget's disease and Indeterminate colitis.

## Patentansprüche

1. Fermentiertes getrocknetes Material, das von der fermentierten Flüssigkeit , die erhältlich ist durch das Fermentieren von Weizenkeimen mit *Saccharomyces cerevisiae* in einem wässrigen Medium, oder Fraktionen davon stammt, zur Verwendung bei der Behandlung einer entzündlichen Darmerkrankung.

2. Fermentiertes getrocknetes Material nach Anspruch 1, wobei die entzündliche Darmerkrankung ausgewählt ist aus Morbus Crohn, Colitis Ulcerosa, kollagener Collitis, lymphozytischer Colitis, ischämischer Colitis, Diversion-Colitis, Behçet-Krankheit und unbestimmter Colitis.

## Revendications

1. Matière séchée fermentée issue du liquide fermenté pouvant être obtenu par fermentation de germe de blé avec *Saccharomyces cerevisiae* dans un milieu aqueux ou des fractions pour une utilisation dans le traitement d'une maladie inflammatoire de celle-ci de l'intestin.

2. Matière séchée fermentée selon la revendication 1, dans laquelle ladite maladie inflammatoire de l'intestin est choisie parmi la maladie de Crohn, la colite ulcéreuse, la colite collagénique, la colite lymphocytaire, la colite ischémique, la colite de diversion, la maladie de Behçet et la colite indéterminée.
